Europäisches Patentamt

European Patent Office

Office européen des brevets

(19)

(11) Publication number: **0 002 300**
**B1**

(12) **EUROPEAN PATENT SPECIFICATION**

(45) Date of publication of patent specification: **20.01.82**

(51) Int. Cl.³: **C 07 D 201/16**
**//C07D207/26**

(21) Application number: **78200321.4**

(22) Date of filing: **29.11.78**

(54) **Process for the recovery of 2-pyrrolidone.**

(30) Priority: **02.12.77 NL 7713349**

(43) Date of publication of application:
**13.06.79 Bulletin 79/12**

(45) Publication of the grant of the European patent:
**20.01.82 Bulletin 82/3**

(84) Designated Contracting States:
**BE DE FR GB**

(56) References cited:
**GB - A - 1 251 258**
**GB - A - 750 222**
**US - A - 4 050 994**

(73) Proprietor: **STAMICARBON B.V.**
**Postbus 10**
**NL-6160 MC Geleen (NL)**

(72) Inventor: **Goettsch, Reijer**
**Aldenhofstraat 15**
**NL-6191 GP Beek (L.) (NL)**
Inventor: **Jetten, Arnold Godfried Maria**
**Rector Thijssenstraat 1**
**NL-6237 NG Moorveld (NL)**

(74) Representative: **Pinckaers, August René et al,**
**OCTROOIBUREAU DSM Postbus 9**
**NL-6160 MA Geleen (NL)**

Courier Press, Leamington Spa, England.

Process for the recovery of 2-pyrrolidone

The invention relates to a process for the recovery of 2-pyrrolidone from a reaction product obtained in the preparation of 2-pyrrolidone by the hydrogenation of succinonitrile and reaction with water of the product formed in this hydrogenation (see e.g., Netherlands Patent Application 7502574 and United States Patent Specification 3,644,402). While this method yields a reaction product from which pyrrolidone of excellent purity can be recovered by fractional distillation, it has however been found, that a significant amount of pyrrolidone is lost in the distillation.

It is known from British Patent Specification 750,222 that lactams can be recovered from a reaction mixture obtained by Beckmann transformation of oximes with a large amount of a strongly acid reagent, by suitable dilution of the reaction mixture with water and subjecting the diluted reaction mixture to an extraction with a chlorinated hydrocarbon.

In British Patent Specification 1,251,258 is disclosed that caprolactam can be recovered from wash water obtained by the treatment of polycaprolactam with water by extracting the wash water, advantageously without previous concentration, with a solvent like chlorinated hydrocarbons and esters of a monohydric alcohol with a monobasic acid.

The invention provides a very suitable process for the recovery of pyrrolidone from the above-mentioned reaction mixture.

The process according to the invention for the recovery of 2-pyrrolidone from a reaction product obtained by hydrogenation of succinonitrile and reaction with water of the product formed in this hydrogenation is characterized in that an aqueous solution of the reaction product is concentrated by evaporation of water and adjusted to a pH of 2—6, the resulting solution is subjected to an extraction with chloroform or methylene chloride to form an organic phase containing 2-pyrrolidone and the 2-pyrrolidone is recovered from this organic phase.

The concentration by evaporation of water in the process according to the invention, e.g. to a concentration of 50—60% by weight, has the advantage that volatile impurities are also removed from the solution. The required pH of the solution can be adjusted by adding acid, e.g. sulphuric acid or acetic acid, or by forming acid in situ in the solution e.g. by passing carbon dioxide through the solution.

The extraction of the aqueous solution can readily be carried out in counter-current flow with the extraction agent at a temperature of e.g. 15—90°C. The extraction is preferably carried out at a temperature between 15° and 45°C, as atmospheric pressure may then be used. If chloroform is used as the extraction agent, a good separation is effected between the impurities in the aqueous phase and the pyrrolidone in the organic phase. Usually 2—20 grams of extraction agent are used for every gram of pyrrolidone to be extracted. If desired the organic phase obtained may be washed with water. The by-products formed in the preparation of the pyrrolidone, e.g. 1,4-diaminobutane, can be recovered from the aqueous phase remaining in the extraction.

The recovery of the pyrrolidone from the organic phase obtained in the extraction may be done in various ways. For instance the solvent may be removed by evaporation or the pyrrolidone may be crystallized out. Preferably the organic phase is subjected to an extraction with water and the pyrrolidone is recovered from the resulting aqueous solution of pyrrolidone by crystallization from the solution or by evaporation of the water. This extraction may be carried out for example with 1 to 8 grams of water per gram of pyrrolidone to be extracted and at a temperature of 15—50°C. If desired the recovered pyrrolidone may be subjected to distillation to achieve the highest possible purity. Hardly any pyrrolidone gets lost in this distillation.

The invention will be further elucidated in the following Examples.

### Example I

A dilute aqueous reaction mixture of pyrrolidone obtained by hydrogenation of succinonitrile and reaction with water of the hydrogenated product in the way described in Netherlands Patent Application 7502574 is concentrated by evaporation of water to form a solution containing 51% by weight of pyrrolidone, 40% by weight of water and 9% by weight of other compounds (among which is 1,4-diaminobutane). The pH of this solution is lowered from its original value of 12 to a value of 5 by adding sulphuric acid (96% by weight) to the evaporated solution. The resulting aqueous solution is then extracted at 25°C in a column in counter-current flow with chloroform. 289 grams of chloroform are used for every 100 grams of aqueous solution. The resulting organic phase contains 15% by weight of pyrrolidone, while substantially no pyrrolidone is contained in the aqueous phase. The extraction efficiency is 99.9%. The organic phase is then extracted in counter-current flow with water at a temperature of 25°C. In this extraction 35 grams of water are used for every 100 grams of organic phase.

The efficiency of this extraction is also 99.9%. The chloroform still contained in the resulting aqueous phase is removed by evaporation, after which an aqueous solution remains that contains 28.9% by weight of pyrrolidone (purity 99%). The diaminobutane originally present can no longer be detected in this aqueous solution. The pyrrolidone is obtained in the solid state by evaporation of the water from the aqueous solution. After distillation of this pyrrolidone (133°C, 12 mm of Hg), its purity is 99.6%. Only 1.7% of the pyrrolidone contained in the product to be distilled gets lost in this distillation.

### Comparative Example

An aqueous reaction mixture similar to that used as a starting product in Example I is subjected to fractional distillation. At 133°C and 12 mm of Hg, a fraction containing pyrrolidone is obtained which has a purity of 99% by weight. The loss of pyrrolidone then amounts to 7% of the original amount in the aqueous reaction mixture.

### Example II

A dilute aqueous reaction mixture of pyrrolidone obtained by hydrogenation of succinonitrile and reaction with water of the hydrogenated product in the way described in Netherlands Patent Application 7502574 is concentrated by evaporation of water to form a solution containing 60% by weight of pyrrolidone, 30% by weight of water and 10% by weight of other compounds. The pH of the solution is lowered from 12 to 5 by means of 96%-by-weight sulphuric acid. The solution is then extracted at 25°C in counter-current flow with 540 grams of methylene chloride for every 100 grams of solution. The extraction efficiency is 99.9%. The resulting organic phase (about 10% by weight of pyrrolidone) is then extracted at 25°C in counter-current flow with 35 grams of water for every 100 grams of organic phase (extraction efficiency 99.9%). Solid pyrrolidone (purity 99%) is recovered by evaporation of the water from the resulting aqueous solution. The purity of the solid pyrrolidone is raised to 99.5% by distillation at reduced pressure (133°C, 12 mm of Hg). The loss of pyrrolidone in this distillation is only 1.4% calculated to the pyrrolidone present in the product to be distilled.

### Claim

A process for the recovery of 2-pyrrolidone from a reaction product obtained by hydrogenation of succinonitrile and reaction with water of the product formed in this hydrogenation, characterized in that an aqueous solution of the reaction product is concentrated by evaporation of water and adjusted to a pH of 2—6, the resulting solution is subjected to an extraction with chloroform or methylene chloride to form an organic phase containing 2-pyrrolidone and the 2-pyrrolidone is recovered from this organic phase.

### Patentanspruch

Verfahren zum Zurückgewinnen von 2-Pyrrolidon aus einem durch Hydrieren von Bernsteinsäurenitril und ein Reaktion mit Wasser des in dieser Hydrierung gebildeten Produkts anfallenden Reaktionsprodukt, dadurch gekennzeichnet, dass eine wässrige Lösung des Reaktionsprodukts durch Verdampfen von Wasser konzentriert und auf einen pH-Wert von 2 bis 6 gebracht wird, die anfallende Lösung einer Extraktion mit Chloroform oder Dichlormethan unterworfen wird, damit eine organische 2-Pyrrolidon enthaltende Phase gebildet wird, und das 2-Pyrrolidon aus der organischen Phase zurückgewonnen wird.

### Revendication

Procédé d'obtention de 2-pyrrolidone à partir d'un produit réactionnel, obtenu par l'hydrogénation de succinonitrile et la réaction avec de l'eau du produit formé lors de cette hydrogénation, caractérisé en ce qu'une solution aqueuse du produit réactionnel est concentrée moyennant l'évaporation de l'eau et portée à un pH de 2—6, que la solution obtenue est soumise à une extraction avec du chloroforme ou du chlorure de méthylène avec formation d'une phase organique contenant de la 2-pyrrolidone, et que la 2-pyrrolidone est obtenue de cette phase organique.